# EUROPEAN PATENT APPLICATION

(11) **EP 4 442 789 A1**
(43) Date of publication of application: **09.10.2024**
(21) Application number: 24020094.9
(22) Date of filing: 28.03.2024
(51) Int. Cl.: C09K 8/582, E21B 43/16

(54) **NOVEL COMPREHENSIVE SCALABLE PROCESS FOR ON-SITE BIOPOLYMER PRODUCTION FOR ENHANCED OIL RECOVERY PROJECTS**

(30) Priority: 30.03.2023 AR P230100781
(71) Applicant: Interenergy Argentina S.A, Ciudad de Buenos Aires (AR)
(72) Inventor: Götz, Pablo, Ciudad de Buenos Aires (AR)
(74) Representative: Ruo, Alessandro

(57) **Abstract**

COMPREHENSIVE, SCALABLE PROCESS FOR ON-SITE BIOPOLYMER PRODUCTION RELATED TO ENHANCED OIL RECOVERY PROJECTS, comprising the following stages: a) Activation-Subculture: Bacterium/fungus (15) activation with the selected substrates (16) by placing the same in a sterile, solid culture medium (17) and incubating it at a certain temperature until reaching the desired polysaccharide production level; b) Shake Flasks: Next, a replication sequence is undertaken in a sterile, liquid culture medium (18) in order to increase the amount of biomass until a laboratory inoculum (19) is obtained; c) Fermentation Vats: Fermentation takes place in fermentation vats or bioreactors (20), where substrates (16) are transformed by microorganisms into biopolymers and biomass; d) Filtration or Centrifugation: Centrifugation (22), which causes the liquid biopolymer (24) to separate from biomass (23); e) the liquid polymer (24) obtained during stage d) is mixed with injection water (44) in a static mixer (45), and then injected directly into the reservoir through an injection well (46). Biopolymer manufacture is performed in the proximity of the reservoir injectors and the supernatant from stage d) filtration or centrifugation is placed directly into the injection line directly to the injectors. The process is initially carried out with a minimal setup at a reservoir site that is close to a site with substrate availability to activate the subculture. The biopolymer is an exopolysaccharide such as xanthan gum and scleroglucan.

## Description

### A. TECHNICAL FIELD OF THE INVENTION

This patent application relates to an oilfield operation method that provides significant advantages resulting, among others, from a smaller number of equipment items required to carry out the process (with the ensuing reduction in maintenance time and tasks), and dispensing with the need to install a polymer hydration plant on the site to have a polymer solution injected into injection wells.

More specifically, this patent application relates to an operation method of the type specified above, which has been designed and developed thanks to the experience gained in oil well development and, more specifically, in enhanced oil recovery operations in such wells, which are injected with water mixed with biopolymer and/or polyacrylamide in order to improve sweep efficiency and reduce the amount of injection water used.

The biopolymer production process for use in enhanced oil recovery that is the subject matter of this patent application substantially reduces biopolymer losses in comparison to the traditional industrial biopolymer production method. Moreover, this new process offers significant savings in terms of facilities, operating costs, and use of nutrients to obtained the required viscosity target.

The major advantage offered by this invention is the reduced initial investment required to start the project (with the ensuing risk reduction) and the fact that, once the method has proven successful in the selected reservoir, the possibility to scale up the investment to massify to the rest of the field.

The biopolymer/water solution offers enhanced sweep efficiency in comparison to using water alone in secondary oil recovery, thus improving the water footprint of the oil extraction process. It should be noted that this is a sustainable process.

### B. STATE OF THE ART AND PROBLEMS TO BE SOLVED

### B.1 Concepts related to Oil Field Development

In conventional oil fields, reservoirs are generally developed by drilling naturally producing wells first. This is called primary recovery.

When reservoir pressure goes down, water is injected into injection wells in order to stimulate the reservoir and sweep trapped oil, a process known as secondary recovery. Reservoir heterogeneity or oil viscosity may often times render this mechanism inefficient. Several pore volumes of water (the measure used to identify reservoir volume) may be injected and produced (i.e., over 50 m³ of water are often times required to obtain 1 m³ of oil). The produced water obtained after oil separation is generally reinjected.

The recovery factor (how much oil has been recovered over a given time period) is a very important factor in oil field development, but it is largely dependent on a series of parameters, including the following:
- Petrophysical Quality of the Reservoir: Porosity, permeability, and relative permeability curves; Oil Type: e.g. API gravity, and viscosity;
- Energies Involved: Dissolved gas expansion, natural aquifer drive, gas cap drive.

Primary oil recovery is estimated to range between 10 and 30 per cent, while the secondary recovery factor is between 5 and 15%. The rest remains in the reservoir, to be subsequently recovered.

Several processes exist, including the so-called enhanced oil recovery, to increase recovery factors and actually extend the life of reservoir operations. Pilot tests are generally carried out in order to decide whether the project should be scaled up to include all of the field/reservoir.

Pilot test refers to a project intended to prove a given technology that is either new in the industry or new for the field/reservoir. The necessary parameters should be present in the pilot project in order to then decide whether the same may be extrapolated or scaled up to include all of the field/reservoir.

Chemical injection to enhance water viscosity is a well-known enhanced (or tertiary) recovery mechanism, thus rendering reservoir drive (and therefore hydrocarbon sweep) more efficient. Ideally, water viscosity should be such that its displacement rate is similar to that of the oil to be displaced. Polymers are used to increase viscosity in most chemical enhanced oil recovery methods.

An anionic polyacrylamide, a polymer consisting of acrylamide monomer that may be combined with other monomers, such as acrylic acid, is used to form copolymers. There are several production processes, the most common of which are copolymerization, co-hydrolysis, and post-hydrolysis. The molecular structure of partially hydrolyzed polyacrylamide is shown below:

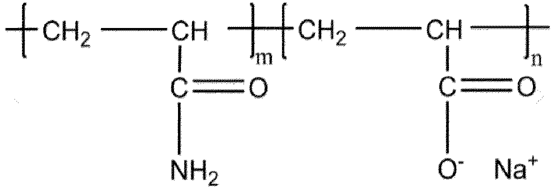

The features of the polymer manufacturing process are as follows:
a) A significant production volume is required for plants to be profitable (generally above 100t/day), hence the limited number of plants existing throughout the world.
b) The specific raw material required for this process is acrylamide monomer, which is obtained from acrylonitrile, in turn produced through propylene ammoxidation (propylene is a byproduct from petroleum refining). These are generally captive raw material markets, where prices are subject to fluctuations in acrylonitrile and petroleum costs.
c) Given the scarce number of plants existing throughout the world, transport costs to bring the product to oil fields are high. Costs (raw material and transport, among others) conspire against lengthy projects, the consequence being that products are injected at lower quantities for long periods of time in order not to discontinue ongoing processes.

In view of the above, process economy is generally not aligned with the economy of the oil to be produced.

Polyacrylamide today is mostly produced in China. The raw material, a byproduct of the petrochemical industry, is trucked to a polyacrylamide production plant in China where, following a polymerization process, the polymer gel is obtained, which is subsequently pelletized, cut, and dried. After drying, pellets are ground to a particle size that is in line with the nature of the polymer and manufacturing specifications, bagged, and trucked to the shipment port. Upon arrival in the destination country, the material is sent to the field, where the polymer is hydrated using fresh or injection water in a special plant (these plants are generally imported). Finally, the product (now with a controlled viscosity level), is injected into injection wells.

### B.2 General Biopolymer-related Concepts:

There are other products which might be used for enhanced oil recovery, one of them being biopolymers. Xanthan gum and scleroglucan are the two most frequently used, but the invention that is the subject matter of this patent application might also be applied to other polymers produced by microorganisms. Xanthan gum is produced by bacteria, while scleroglucan is produced by fungi.

A biopolymer is a macromolecule that is synthesized via biological processes. Biopolymers may be resourced from several natural sources, including plant exudates, seaweed extracts, seeds, plant extracts, animals and microbes.
a) Xanthan gum (XG): It is a hetero-polysaccharide formed by monomer units that contain glucose, mannose, and glucuronic acid. Xanthan gum is produced by bacteria (*xanthomonas campestris*). As shown below, Xanthan gum may be found in a double helix structure, with side branches facing outwards: 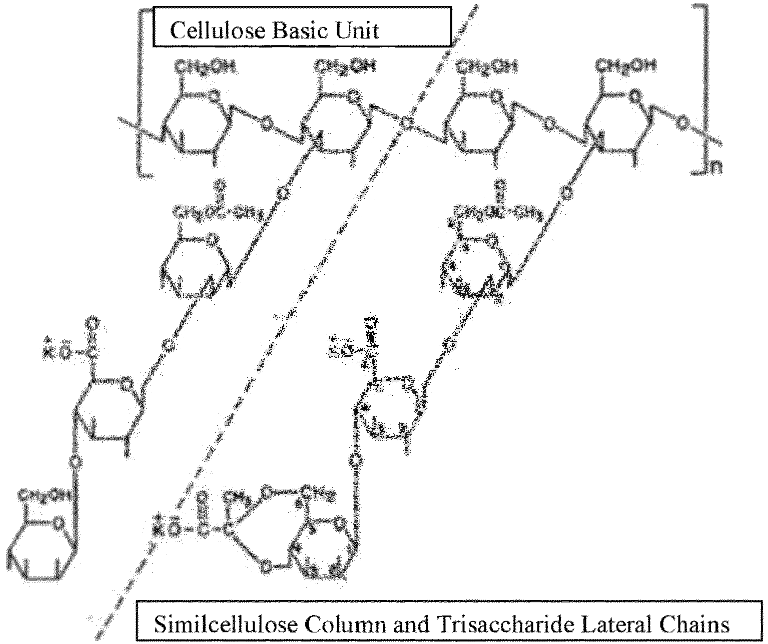
b) Scleroglucans (SG): Non-ionic polysaccharide formed by beta-D-glucopyranosyl units. In the solution form, it shows a triple-chain helix structure linked by hydrogen bonds. It is produced by several species of the *Sclerotium* fungus. The structure (A) and 3-D conformation (B) of scleroglucan are shown below.

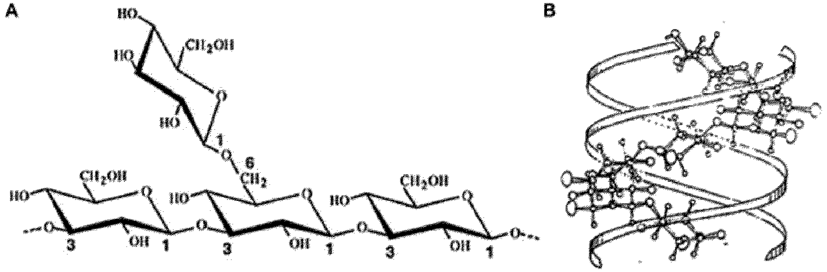

Polyacrylamide and/or biopolymer injection together with water delivers a significant positive effect by improving sweep and therefore reducing to less than one third the use of injection water. This in turn provides significant energy savings, since less water is used for injection/production, while also improving the process water footprint, given that less water is used per unit of recovered oil.

**In comparison to polyacrylamides, biopolymers offer the following advantages:**
a) Ionic Stability: Since this is a non-ionic polymer, it offers a high tolerance in a wide range of salinities, and it may therefore be used in a reservoir with a higher salinity level in comparison to conventional polyacrylamide.
b) Mechanical Stability: In a solution form, it has a semi-rigid structure, with minimum branching. Biopolymers do not degrade as much as a synthetic linear polymer when going through the valves or porous medium, as they are more resistant to shear forces. This becomes particularly relevant in oil fields where multiple reservoirs are injected, since this would in several instances allow multiple zone completion and conventional selective facility type.
c) Thermal Stability: Absence of easily hydrolysable groups. For example, SG thermal aging studies have shown an initial viscosity level maintained at 100°C for two years. In comparison to conventional polyacrylamide, it may be applied to higher-temperature reservoirs.
d) Higher Viscosity Development: Biopolymers manage to develop 3 to 4 times higher viscosity levels when compared to polyacrylamide at identical concentrations. This means using less product to generate a given target viscosity.
e) Chemical stability in the presence of H₂S and Fe⁺⁺ in aerobial conditions. It allows reducing the use of chemicals such as oxygen sequestrants, free radicals or other sacrificial agents.
f) Once the microorganism has been identified, no complex raw material is required for biopolymer manufacturing, which is a simple, environment-friendly process.

**Polyacrylamide offers the following advantages in comparison to biopolymers:**
a) It is less prone to attack by natural bacteria present in formations and systems. Despite of that, polyacrylamide is very susceptible to chemical degradation via some bacteria by-products, so microbial control must be also used.
b) Given the manufacturing process used, the viscosity is more replicable.
c) It offers better controlled filterability, as well as easier dilution and hydration than powder biopolymer.

Even with this comparison, which clearly favors biopolymers, these are hardly injected today for enhanced oil recovery for the following main reasons, namely:
1. Very high costs associated to the facilities, high operating costs, purchase of nutrients and other supplies, and significant loss of biopolymer along the purification process, i.e., financial reasons that render it non-viable.
2. Additionally, since it is a biological product, it is difficult to supply a product for this kind of process that both meets customer's specifications and remains stable throughout time. Dispersibility and filterability are some of the weaknesses identified as far as quality is concerned.
3. There is high demand for these products by other industries, including foodstuffs, cosmetics and medicinal drugs, among others, so ongoing supply may not be guaranteed.
4. Given the implicit risk of not achieving the desired viscosity level in the field and of the reservoir not meeting the expected technical result (financially speaking), it is not possible to enter into long-term supply agreements. All of the above means that, given the high cost involved, resorting to the pilot project method often proves inefficient and non-feasible.

The significant distances separating biopolymer manufacturing plants and fields increase logistics costs. Also, hydrating the product makes this a costly investment. In summary, even though short-lived projects were undertaken twenty years ago, the main problem associated to this kind of product is the high cost of buying products offering a stable quality. Consequently, and despite its advantages when it comes to sustainability, the reasons listed above deter these projects and this method is therefore not widely used.

### B.3 Concepts related to Biopolymer Manufacturing.

### B.3.1 Traditional Method

### Laboratory Determination prior to Large Scale Manufacturing:

a) The first step in the process consists in developing and adjusting product formulation. A possible work flow consists in conducting a low-scale "screening" in shake flasks and lab-scale bioreactors in order to assess strain productivity using standardized means and varying availability of carbon sources, and then characterize the resulting biopolymers (e.g. viscosity and filterability, among other features). The suggested approach in this stage is to assess potential sources of alternative substrates for culture medium formulation purposes, taking into account the quality, commercial value and availability of the selected substrate(s) in the local market, with a focus on the circular economy of value chains related to the sources of the carbon to be used in biopolymer production. The strain, culture medium and biopolymer offering the highest viscosity (centipoise per gram per liter) are selected. The kinetics of each production is assessed. High product/substrate yields, low biomass production, increased production over shorter process times, and low substrate residue in final fermented medium are desirable.
b) Scaling up to bioreactors: Once the product(s) has/have been selected, downstream variables determining end-product rheology (strain, inoculation, culture time and conditions) are optimized. Next, the process is tested in larger bioreactors to reach the pilot scale. Viscosity, molecular weight and conformational parameters are usually measured.
   1) The traditional biopolymer large scale manufacturing process and subsequent use in enhanced oil recovery are described below:
      2) ***Activation-Subculture:*** Once the optimal bacteria and/or fungi strains, together with the selected substrate(s) have been identified at the lab, the bacterium/fungus is activated, placed in a sterile, solid culture medium, and incubated at a given temperature until reaching the desired polysaccharide production level.
      3) ***Shakers:*** Next, a replication sequence is undertaken in a sterile, liquid culture medium in order to increase the amount of biomass until a laboratory inoculum is obtained.
      4) ***Fermentation:*** Next, the fermentation process itself takes place. This is a catabolic reaction where energy-releasing complex molecules result in simple molecules. The process whereby complex carbohydrates turn into simple molecules used by the microorganism to produce biomass and other molecules generates carbon dioxide. Fermentation takes place in fermentation vats or bioreactors, where substrates (nutrients) are transformed by microorganisms into metabolites (biopolymers) and biomass. Fermentation is an incomplete oxidation process, the end result of which is an organic product. Fermenters of successively larger sizes are used. By way of example, a 10x rate may be used (i.e., start with 5 liters, to then move on to 50, 500, 5,000, 50,000, and 500,000 liters). This is where the upstream stage ends. The culture medium needs be sterilized in all steps of the process.
      5) ***Biomass Separation***: This constitutes the beginning of the so-called downstream stage. As a first step of the pure product recovery process, biomass is separated from water-soluble components making up the fermentation liquor. A suitable method consists in diluting (and sometimes adjusting the pH of and/or heating) the fermentation liquor using a sufficient amount of water in order to reduce viscosity and facilitate polymer-biomass separation during the filtration or centrifugation stage.
      6) ***Precipitation:*** Precipitation agents such as methyl, ethyl or propyl alcohol or ketone are generally used, normally at a temperature above 100°C. Impurity removal may be done by using the same solvent, or by dissolving the polysaccharide in water and precipitating the same again. This process may be repeated up to 3 or 4 times, thus obtaining the biopolymer with the desired purity level. Precipitation and impurity removal generally constitute the highest operating cost in the downstream stage.
      7) ***Drying:*** Following the precipitation step, the product is dried through filtration, precipitation or centrifugation. Prior to that, the filtered/centrifuged product must be dried using air, ketone, spray, or a rotary vacuum-drum filter.
      8) ***Grinding and Packaging:*** The resulting product (generally white and fibrous) is crushed or ground until obtaining the desired granulometry. It is convenient to dry or dehydrate the same as much as possible in order to reduce the amount of water it may convey.
      9) ***Transportation:*** The dry product is carried from the manufacturing plant to the fields in containers of a suitable size and material.
      10)***Hydration Plant:*** A hydration plant is used in oil fields, where the biopolymer dose is formulated in order to obtain the target viscosity level with injection water.
      **11)Well *Injection:*** Once the water with the necessary viscosity level has been obtained, it is injected into injection wells.

### B.3.2 Factors to be taken into account in biopolymer manufacturing

Main factors playing an important role in biopolymer manufacturing are as follows:
a) ***Nutrients:*** The substrate is the main raw material together with the fungus/bacteria, so this is a very important factor for project economy. The substrate source is diverse and, given the characteristics of the current invention, it is very important that it be located near the oil field. Sources include, by way of example: Sugarcane, sugar beet, honey, corn, etc. There are several byproducts available that may also be used, such as sugarcane molasses, the waste product obtained from the final sugar crystallization process, from which no additional sugar may be obtained using physical methods.
   Glucose and saccharose are the two nutrients which are most frequently used as a carbon source, as they offer the highest yields. Substrate source and structure have a decisive impact on microorganisms' ability to survive and perform the desired function. Whenever any of the substrates offers low water solubility, bioavailability is limited, so the biochemical reaction will be delayed due to a mass transfer issue. Compound concentration (both reagents and products) is also very important. By way of example, a high concentration of a given compound may prove toxic and inhibit growth.
b) ***Concentrations in Culture Medium:*** Carbon sources may range from mono- and disaccharides (e.g. mannitol, glucose, fructose, and sucrose) to organic acids (such as citrate and succinate, among others). It is necessary to find the optimal carbon source concentration (in g/l) that may offer the highest viscosity level. The highest biopolymer concentration (g/l) is not necessarily indicative of a higher viscosity. It is also possible to add other elements in smaller amounts, such as a mixture of calcium nitrate and yeast extract, as well as other micronutrients, to achieve product synergies.
c) ***Product Viscosity Target:*** Product weight and quality are the most important factors in current processes. In the case of enhanced oil recovery using biopolymers, the most important factor is the target viscosity generated by the product considering the conditions prevailing in injection water. Thus, generating a larger amount of product (by weight) does not equate a better economy related to the target viscosity level per m3 of injected water.
d) ***Biopolymer Loss:*** Approximately 50% of biopolymer produced in processes 4 through 7 above is lost due to inefficiencies in the traditional process.
e) ***Escalation:*** In the escalation from the lab to the industrialization stage, the similarity law is applied, which uses parameters such as geometry, dynamics, energy transfer, mass transfer, and chemical reactions.
f) ***Time:*** The time taken to develop a fungal/bacterial colony and produce the biopolymer. This period of time (usually 48 to 72 hours) will then be linked to the required facilities. Time is of the essence, as it materially conditions the size of process facilities that will be required.
g) ***pH:*** pH has a significant impact on microbial activity. Each microorganism has a pH range within which growth is possible, and, normally, a well-defined optimal pH level.
h) ***Temperature:*** Temperature is one of the most important environmental factors affecting microorganism growth and survival. It is necessary to identify what that optimal temperature is (generally around 30°C), so as to obtain the maximum exopolysaccharide production.
i) ***Aeration and Mixing:*** These are two decisive factors in biopolymer manufacturing, especially at a large scale, where difficulties not seen at laboratory level arise since the surface volume ratio and hydrostatic pressure change when the equipment size is increased. As a result, oxygen transfer taking place through the liquid surface is very important in a small fermentation vat, but almost negligible in large scale equipment. As far as hydrostatic pressure is concerned, bottom pressure is high in industrial fermentation vats, whereas the pressure difference between the surface and the bottom is almost negligible in those used in the laboratory. In view of the above, gases such as CO₂, generated by fermentation, increase their partial pressure at the bottom of fermentation vats where hydrostatic pressure is high, resulting in major changes in process performance and in the molecular features of the biopolymer being manufactured. As far as mixing is concerned, one of the most important aspects of fermentation escalation is the choice and use of suitable impellers. The optimal one should offer the highest possible homogeneity.

### B.3.3 New Comprehensive Process

The invention that is the subject matter of this patent application is a comprehensive, scalable process for on-site biopolymer production related to enhanced oil recovery projects. It is based on a new process to manufacture and use the byproduct which comes to solve most of the technical problems described above, namely:
*1.* ***Reduction in the Number* of *Process Stages:*** The process that is the subject matter of this patent application is reduced to the first four steps of the traditional large-scale biopolymer manufacturing process. This new process results in significant savings in terms of facilities, operating costs, and nutrient consumption required to obtained the required viscosity level.
*2. **Reduced Nutrient Use:*** Nutrient-related savings are a result of the reduction in biopolymer loss in the "downstream" stage and of the search for optimal viscosity levels for injection water and general conditions in selected injection wells (i.e., using process parameters), thus achieving the required viscosity using continuous injection water.
*3. **Installation of a Biopolymer Plant at the Injection Site:*** Polymer manufacturing plants are set up within the oil fields. Only the nutrients and other minor raw materials are carried to the site. Ideally, substrates located near the field should be identified.
*4. **Hydration Plant is no longer required:*** The end product is an aqueous solution ready to be diluted in injection water at the target viscosity level, so dry polymer hydration is no longer required. It may be said that much of the savings obtained from this are used to build the biopolymer manufacturing equipment.
5. ***Scalable Process:*** Scalability is a very important advantage of the process that is the subject matter of this patent application, which requires only a minor investment, so that a limited loss would be incurred if the project fails due to reservoir-related or environmental issues. By way of example, if two injectors are initially used, the project could then be scaled up to 5, 50, 500, 5000 and 50,000 liters, respectively. Should the project work as planned, larger fermentation vats could then be added until completing 500,000 liters, so as to deliver the product to twenty or more injection wells, i.e., the project may be scaled up to the extent results are obtained, thus minimizing the risk.

### C. PURPOSE OF INVENTION

The purpose of this invention is to provide an oil well development method to be used in an oil field that comprises an on-site biopolymer manufacturing process and subsequent injection thereof into the wells, which process consists of the following steps or stages:
*1) **Activation-Subculture:*** Steps or stages are the same as in the traditional biopolymer production process.
2) ***Shakers:*** Steps or stages are the same as in the traditional biopolymer production process.
*3) **Fermenters:*** Steps or stages are the same as in the traditional biopolymer production process.
4) ***Biomass Separation:*** Steps or stages are the same as in the traditional biopolymer production process.
5) ***Well Injection:*** Once the water with the necessary viscosity level has been obtained, it is injected into injection wells.

This eliminates steps 5 through 7 of the traditional, well-known downstream biopolymer manufacturing process, as well as the need to transport dry biopolymer and use a hydration plant to inject the same as a solution (steps 8 and 9). The filtered or centrifuged product (supernatant) obtained in step 4 is mixed with injection water and injected directly into the well. Viscosity level is controlled by regulating it with injection water so as to ensure it is adequate for the project.

Notwithstanding the above stages, transport of nutrients and other minor raw materials to the field, as well as biomass handling, are also two required steps. The medium is sterilized using steam injection and/or UV light.

As noted above, it is possible to start with a minimum number of fermenters and other facilities and then readjust the process economy as required, e.g., start with a 5-I container, to then successively scale up the process to 50, 500, 5,000 and 50,000 liters. The minimum scale allows accommodating one or two injection wells with a capacity of 100 m3/day each and a viscosity of 100 cp.

Specifications related to the water to be used to fill the fermentation vats (e.g. the use of injection water from the field) must be validated at a laboratory scale.

Different fermentation strategies such as fed-batch- may be used in order to optimize the use of facilities.

As far as the filtration or centrifugation stage is concerned (used for biomass separation), and depending on the efficiency of the separation method used, pre-dilution, shearing and homogenization, as well as increased temperature and pH adjustment, may be required to improve biomass/biopolymer separation.

It is important to previously determine process parameters required to obtain a biopolymer with optimal properties for enhanced oil recovery, especially the viscosity of the biopolymer aqueous solution, which maximizes the oil volume being recovered.

The initial stage of the process consists in a pilot test with a baseline configuration that allows producing a certain amount of biopolymer, adjusting process parameters until obtaining the optimal viscosity level. Once this has been achieved, the process is generally scaled up in one or more stages using a progressively larger number of fermentation vats or bioreactors that may generate changes in the end viscosity, with new adjustments in process parameters being required in order to optimize the same.

When the expected result has been achieved in terms of both viscosity and reservoir, the number of fermentation vats is increased so as to engage in mass on-site biopolymer production on the field in an amount sufficient to continuously supply the wells, mixing the supernatant with injection water and injecting the diluted aqueous biopolymer solution directly into the wells. This stage may be conducted at a "satellite" injection well level (1 or 10 or 100 injection wells) or directly at such whole oil field scale. It is a completely scalable process, and so is the investment required to carry it out. One approach consists in setting up the equipment required during the pilot stage in movable containers, so that they may be relocated to various sites throughout the oil field, as necessary.

This new process stands out for the very low initial investment required to start the project and, depending on the results, the pilot phase may be used to optimize the process and/or move on directly to the large-scale production phase, thus significantly reducing costs and risks. This process will clearly allow for quickly implementing these enhanced oil recovery projects.

In conclusion, the invention that is the subject matter of this application introduces a highly viable process to "boost" the development of enhanced oil recovery projects that are not dependent on imported raw materials, thus minimizing risks, costs and the required investment. Additionally, the project is 100% sustainable, and it materially reduces both carbon and water footprints.

### D. SUMMARY DESCRIPTION OF DRAWINGS

In order to facilitate understanding of the present invention, described below is a preferred embodiment that is illustrated schematically but not to scale in the attached drawings, which are included merely by way of explanation and illustration of the basic design underlying the same.
- Figure No. 1 presents a schematic view of recovery mechanisms in conventional oil extraction.
- Figure No. 2 presents a schematic view of the conventional enhanced oil recovery process using biopolymer.
- Figure No. 3 presents a schematic view of water dispersed powder polymers using a polymer injection plant.
- Figure No. 4 presents the enhanced oil recovery process with on-site biopolymer production and direct supernatant injection in accordance with the present invention.

### A. DETAILED DESCRIPTION OF THE INVENTION

In all figures included herein, reference numbers correspond to the same or equivalent number used to identify constituting parts or elements in the example selected to explain the oil well development method that is the subject matter of this patent application.

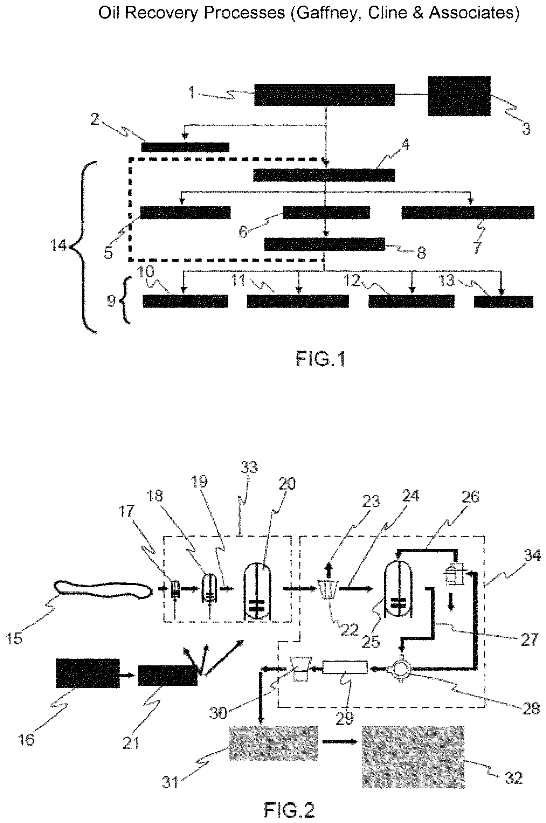

As shown in Figure No. 1, recovery mechanisms in conventional oil recovery comprise primary recovery 1 which relies upon the use of natural energy 2 present in the reservoir. This mechanism also involves naturally flowing gas, water and solution gas 3.

Secondary recovery 4 (consisting in water or gas injection into the well) is carried out when natural energy present in the reservoir decreases. This stage involves drilling infill wells 5, water flooding 6, and maintaining pressure 7.

Finally, tertiary recovery 8 involves the use of methods included in the so-called enhanced oil recovery (EOR) 9. Methods used include those involving thermal 10, miscible flooding 11, chemical 12 and other recovery methods 13, such as microbial EOR (MEOR).

Improved oil recovery (IOR) 14 includes secondary 4 and tertiary 8 oil recovery. Figure No. 2 presents a traditional enhanced oil recovery sequence using biopolymer, which involves the following stages:
1. Activation-Subculture: Once the optimal bacteria and/or fungi strains (i.e., the microorganisms identified by reference No. 15), together with the selected substrates 16 have been identified, the bacterium/fungus is activated, placed in a sterile, solid culture medium 17, and incubated at a given temperature until reaching the desired polysaccharide production level.
2. Shakers: Next, a replication sequence is undertaken in a sterile, liquid culture medium 18 in order to increase the amount of biomass until a laboratory inoculum 19 is obtained.
3. Fermentation vats: Fermentation takes place in fermentation vats or bioreactors 20, where by the action of microorganisms' substrates 16 (nutrients) are transformed into metabolites (biopolymers) and biomass. Fermentation vats 20 of successively larger sizes are used. By way of example, a 10x rate may be used (i.e., start with 5 liters, to then move on to 50, 500, 5,000, 50,000, and 500,000 liters). This is where the upstream stage ends. Culture medium sterilization 21 is required in all steps of the process.
4. Biomass Separation through Filtration or Centrifugation: The centrifugation 22 step marks the beginning of the so-called downstream stage. As a first step of the pure product recovery process, biomass 23 is separated from water-soluble components making up the fermentation liquor. A suitable method consists in diluting, homogenizing and sometimes adjusting the pH of and/or heating the fermentation liquor using a sufficient amount of water in order to reduce viscosity and facilitate polymer 24-biomass 23 separation during the filtration or centrifugation stage.
5. Precipitation: A precipitating agent 26 is used during the precipitation 25 stage to separate the biopolymer 24, normally at a temperature above 100°C. Impurity removal may be done by using the same solvent, or by dissolving the polysaccharide in water and precipitating the same again. This process may be repeated up to 3 or 4 times, thus obtaining the biopolymer 27 with the desired purity level.
6. Drying: Following precipitation 25, the biopolymer 27 undergoes a filtration, settling and centrifugation 28 process. Prior to that, the filtered/centrifuged biopolymer 28 must be dried 29 using air, ketone, spray, or a rotary vacuum-drum filter.
7. Grinding and Packaging: The resulting product (generally white and fibrous) is crushed or ground 30 until obtaining a polymer 31 with the desired granulometry. It is convenient to dry or dehydrate the same as much as possible in order to reduce the amount of water it may convey.
8. Transportation: The dry product -polymer 31- is carried from the manufacturing plant to the oil fields 32 in containers of a suitable size and material.

The "*upstream*" 33 and "*downstream*" 34 stages are identified using dotted lines.

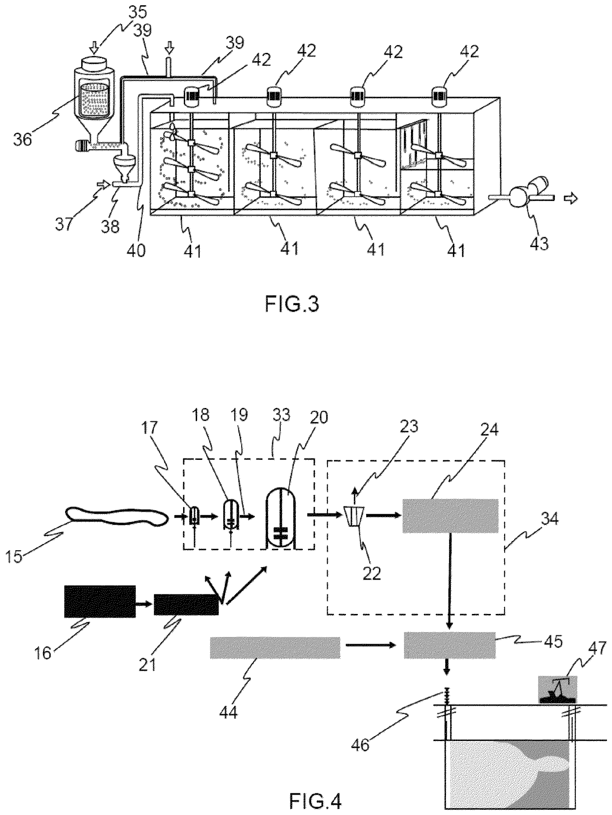

Figure No. 3 presents a schematic view of water dispersed polymers or biopolymers using one plant. The polymer granulate 35 kept in the silo 36 is dissolved and dispersed in water 37 and then injected into the line 38. Nitrogen is injected through the lines 39, and the hydrated polymer circulates along the pipeline 40 to the maturation tank 41, which contains four mechanical agitation stages, driven by their pertinent motors 42. Finally, the pump 43 drives the hydrated polymer solution to the injection wells.

Figure No. 4 presents the biopolymer manufacturing production process for use in enhanced oil recovery in accordance with the present invention. The process comprises the following stages:
1. Activation-Subculture: Steps or stages are the same as in the traditional biopolymer production process. Reference numbers are the same as those used in Figure No. 2.
2. Shakers: Steps or stages are the same as in the traditional biopolymer production process. Reference numbers are the same as those used in Figure No. 2.
3. Fermenters: Steps or stages are the same as in the traditional biopolymer production process. Reference numbers are the same as those used until the fermentation vat 20 section, where a baseline configuration will be used to test and adjust the technology. By way of example, it starts with 5 liters, to then pass on to 50, 500, and 5000 liters, a minimum scale that is suitable for one or two injection wells. The medium is sterilized using steam injection and/or UV light. Use of injection water to fill the fermentation vats must be validated in the laboratory. Different fermentation strategies (such as batch-fed bed) may be used in order to minimize the use of facilities.
4. Biomass Separation through Filtration or Centrifugation: Biomass 23 separation at this reduced scale is the same as in the traditional process, and it may also require dilution in injection water and/or shear and/or heat and/or some pH adjustment.

The biopolymer supernatant 24 obtained in stage 4 is mixed with injection water 44 in a static mixer 45 and injected directly into the reservoir through the injection well 46. Viscosity level is controlled to ensure it is adequate for the project, thus eliminating stages 5 through 8 of the traditional process. Biomass 23 is disposed of or else used as energy or transformed into other products of interest.

The oil producer is identified by number 47. The "*upstream*" 33 and "*downstream*" 34 stages are identified using dotted lines.

The purpose is to deliver biopolymer to a number of injection wells in an enhanced oil recovery project, depending on the equipment size and desired viscosity level. The project is developed as a first pilot test that can then be scaled up to include the rest of the oil field.

***In this first, pilot stage***, a line is built (consisting of fermentation vats and other equipment items) to produce the inoculum required to obtain a fermented supernatant-equivalent daily biopolymer production for subsequent injection into one or two injection wells. It is possible to use laboratory equipment mounted on a trailer, then adding main and ancillary facilities that will be subsequently scaled up as the project is further developed.

Most equipment items may be mounted onto a trailer in order to have a movable laboratory and pilot plant.

The microbiology lab equipment should consist of the following items:
- A freezer to store the starters to be used for biopolymer production; a laminar flow work bench for replication and inoculation at a laboratory level; an incubator for Petri dish microorganism culture from which the inoculum for Erlenmeyer flasks is obtained (from a solid culture medium to a first liquid culture medium).
- Autoclave.
- A system to obtain water that is suitable for broth culture preparation.
- Orbital shakers for culture in Erlenmeyer flasks. Considering, for example, an external reactor with a capacity of 50,000 liters, fermentation would start with five 100 ml Erlenmeyer flasks as a first step of inoculum preparation in a shake flask system, and a 5-liter bioreactor for inoculum preparation.

***By way of example***, during the escalation stage it might be possible to add such ancillary facilities and fermentation vats as required to produce a polymer amount equivalent to eight-ten additional injection wells. The fermentation vat size is gradually scaled up, one phase at a time, starting with a pilot bioreactor (e.g. 50 liters) and then successively increasing the capacity to 500 and 5,000 liters.

Fermentation vat construction details are as follows:
- Cylindrical stainless-steel tank.
- Shaker with top motor, central shaft, two or more blade sets located at different heights along the shaft, suitable for high-viscosity fluids. Standard working speed.
- Internal baffles to avoid imperfect mixing.
- Temperature sensor to maintain temperature at a certain level throughout the fermentation process.
- Dissolved oxygen sensor.
- pH sensor.
- Aeration system to ensure the necessary air volume throughout the fermentation process, measured in vvm, with fine distributed bubbles being generated from the bottom of the fermentation vat (given it is an aerobial fermentation process). Air is injected into the bioreactor through bubbles, which constitute the gas-liquid mass transfer medium. Bubbles exit the bioreactor through diffusers located beneath the impeller. Turbine-like impellers are constantly turning, and the bubbles exiting the diffuser result in significant gas dispersion inside the vessel.
   - Air-tight seal.
   - Temperature control system (casing).
   - CIP system.
   - Non-porous TIG welds.
   - Entrance/inspection manhole.
   - Connector outlet with air exhaust filter.

Self-made external reactors (cylindrical stainless-steel tanks) with a total capacity of 50,000 liters, for example. Size will be a function of the amount of water and the conditions under which it must be viscosified, as well as of the target viscosity level.

The bioreactor cycle will depend on the available volume and pumps. An example would be as follows:
Line feeding and sterilization: 2 hours
Inoculation and fermentation: 72 hours
Emptying: Defined as per the ongoing supply
Cleaning: 1 hour

The biopolymer dilution stage is carried out on line, as the biopolymer is injected into the well (depending on the end viscosity required for the reservoir). With a view to facilitating the fermentation process and abiding by production cycles, there must be at least two bioreactors (50,000 liters each, for example) working in parallel, so that while one is in the fermentation stage the other serves as a surge tank, alternatively using one or the other to dilute and inject the biopolymer.

Before being injected into the well, the fermented broth is centrifuged to separate the biomass, to which end tubular basket centrifuges may be used. At least two centrifuges are used, alternating between one and the other in order to ensure continuous operation, since the centrifuge must be stopped in order to remove the biomass.

At least one mixer is required to dilute the centrifuged fermentation supernatant with injection water until obtaining the desired viscosity level. The supernatant is fed from the surge tank. In a 1:3 dilution ratio, for example, the diluted solution flow is as follows:
Centrifuged supernatant: 1x l/h
Dilution water: 2 x l/h
Volume to be injected: 3x l/h

## Claims

1. COMPREHENSIVE, SCALABLE PROCESS FOR ON-SITE BIOPOLYMER PRODUCTION RELATED TO ENHANCED OIL RECOVERY PROJECTS, **characterized by** the following stages: a) Activation-Subculture: Bacterium/fungus (15) activation with the selected substrates (16) by placing the same in a sterile, solid culture medium (17) and incubating it at a certain temperature until reaching the desired polysaccharide production level; b) Shake Flasks: Next, a replication sequence is undertaken in a sterile, liquid culture medium (18) in order to increase the amount of biomass until a laboratory inoculum (19) is obtained; c) Fermentation Vats: Fermentation takes place in fermentation vats or bioreactors (20), where substrates (16) are transformed by microorganisms into biopolymers and biomass; d) Filtration or Centrifugation: Centrifugation (22), which causes the liquid biopolymer (24) to separate from biomass (23); e) the liquid polymer (24) obtained during stage d) is mixed with injection water (44) in a static mixer (45), and then injected directly into the reservoir through an injection well (46).

2. COMPREHENSIVE, SCALABLE PROCESS FOR ON-SITE BIOPOLYMER PRODUCTION RELATED TO ENHANCED OIL RECOVERY PROJECTS in accordance with claim 1, **characterized by** the fact that the method is initially applied using a baseline configuration in a given site within the oil field.

3. COMPREHENSIVE, SCALABLE PROCESS FOR ON-SITE BIOPOLYMER PRODUCTION RELATED TO ENHANCED OIL RECOVERY PROJECTS in accordance with claims 1 and 2, **characterized by** the fact that the site is close to one where the substrate required to activate the subculture is available.

4. COMPREHENSIVE, SCALABLE PROCESS FOR ON-SITE BIOPOLYMER PRODUCTION RELATED TO ENHANCED OIL RECOVERY PROJECTS in accordance with claims 1 and 3, **characterized by** the fact that the substrate used to activate the subculture is molasses and/or glucose and/or sucrose obtained from sugar cane or another crop.

5. COMPREHENSIVE, SCALABLE PROCESS FOR ON-SITE BIOPOLYMER PRODUCTION RELATED TO ENHANCED OIL RECOVERY PROJECTS in accordance with claim 1, **characterized by** the fact that the biopolymer is an exopolysaccharide such as but not limited to Scleroglucan and Xanthan gum.

6. COMPREHENSIVE, SCALABLE PROCESS FOR ON-SITE BIOPOLYMER PRODUCTION RELATED TO ENHANCED OIL RECOVERY PROJECTS in accordance with claim 1,**characterized by** the fact that it comprises manufacturing the biopolymer near the oil field injection wells and directly injecting the supernatant obtained during stage d) (filtration or centrifugation) into the injection line (injection wells).
